# EUROPEAN PATENT SPECIFICATION

(11) **EP 1 023 090 B1**
(45) Date of publication and mention of the grant of the patent: **22.01.2003**
(21) Application number: 98950114.3
(22) Date of filing: 10.10.1998
(51) Int. Cl.: A61L 27/00

(54) **LUBRICIOUS COATING FOR MEDICAL DEVICES**
GLEITMITTELBESCHICHTUNG FÜR MEDIZINISCHEN VORRICHTUNGEN
REVETEMENT LUBRIFIANT POUR DES DISPOSITIFS MEDICAUX

(30) Priority: 15.10.1997 US 62363 P
(43) Date of publication of application: 02.08.2000
(73) Proprietor: Tyco Group S.A.R.L., 2420 Luxembourg (LU)
(72) Inventor: HUANG, Yeong, Hua, St. Louis, MO 63146 (US)
(74) Representative: Blum, Rudolf Emil Ernst
(86) International application number: EP9806432
(87) International publication number: WO99019004

(56) References cited:
- WO-A-96/39204
- US-A- 4 589 873
- US-A- 5 290 585
- US-A- 5 295 978
- US-A- 5 348 678
- US-A- 5 776 611

## Description

### Field of the Invention

The present invention relates to a lubricant coating for medical devices, and more particularly, to a hydrophilic polymeric coating composition which aids medical devices to become slippery when wetted. The composition of the present invention may be employed as a lubricant coating to reduce the coefficient of friction of catheters, arterial venous shunts, gastroenteric feed tubes, endotracheal tubes and other medical implants or polymeric substrates. Methods are also provided for the manufacture of the subject lubricant coating and for the application of the same to surfaces of medical devices.

### Background of the Invention

Known lubricant coatings applied to surfaces of medical devices include coatings of polyvinylpyrrolidone, polyethylene oxide, hyaluronic acid and its salts, polyacrylic acid and its derivatives, polyurethane, acrylic polyester, fluorocarbons, silicone, and combinations of these substances. For example, Micklus et al., U.S. Patent Nos. 4,100,309 and 4,119,094 relate to a hydrophilic coating of polyvinylpyrrolidone-polyurethane interpolymer formed using polyisocyanate. Ratner et al., U.S. Patent No. 3,939,049 relates to a method of grafting hydrogels for lubrication to polymeric substrates using radiation. Hungton et al., U.S. Patent No. 3,975,350 relates to hydrophilic polyurethane polymers for use as lubricants. Storey et al., U.S. Patent No. 3,987,497, relates to a tendon prothesis having a lubricant hydrogel coating. Many known lubricious coatings are prone to various disadvantages when used in the medical field. Disadvantages of such known lubricants may include insufficiently low coefficient of friction, lack of permanence and hydrophilicity such as characteristic of silicone- or fluorocarbon-based coatings, slipperiness when dry as well as wet thus making handling difficult, utilization of hazardous solvents in the manufacture of the same and utilization of unstable reactive materials in the manufacture of the same. Lubricants produced for medical use from unstable reactive material often require the coating solution to be prepared daily or more frequently to be useful and thereby increases waste and expense. Lubricants produced for medical use involving hazardous solvents are undesirable due to patient toxicity concerns and OSHA considerations.

In order to solve these and other potential disadvantages of known lubricants such as those of the above-cited patents, a lubricant coating is needed which when wetted has sufficient lubricity to be useful in the medical device field such as for medical implants. The lubricant coating must be capable of adhering to a wide variety of substrates and resist wet abrasion. It would also be desirable to have such a lubricant coating prepared from chemically stable and biocompatible solvents.

### Summary of the Invention

The present invention provides a lubricant coating composition comprising an alkylbenzene selected from the group consisting of toluene, xylene and styrene, a hydrophilic polymer selected from the group consisting of polyvinylpyrrolidone, polyvinyl alcohol, polyacrylic acid or a derivative thereof, hyaluronic acid or a salt thereof and polyethylene oxide, an isocyanate-terminated prepolymer selected from the group consisting of polyoxyethylene-based isocyanate prepolymers and an alkylester of a carboxylic acid selected from the group consisting of ethyl lactate, methylbenzoate and propyl acrylate. The present invention also provides a method of making the subject lubricant coating composition which adheres to a wide variety of substrates and resists wet abrasion. The subject lubricant coating composition is chemically stable and is biocompable as described in greater detail below.

A method for using the subject lubricant coating composition to coat medical devices is provided herein which involves cleaning or washing, drying, dip coating or applying of the lubricant, air drying or removal of excess lubricant, optionally baking and packaging a medical device either before or after sterilization thereof.

The present invention also provides a medical device whereby at least a portion thereof is coated with the subject lubricant coating composition which is characterized as being able to achieve a wetted lubricity with a reduction of friction of more than fifty (50) percent.

### Detailed Description of the Invention

The composition of the present invention has been found particularly useful as a lubricant coating in lowering the coefficient of friction of medical devices such as indwelling thoracic catheters and other medical devices. The subject coating is manufactured from a blend of one or more alkylbenzenes selected from toluene, xylene, or styrene but preferably toluene to increase stability, an alkylester of a carboxylic acid selected from ethyl lactate, methylbenzoate, or propyl acrylate wherein ethyl lactate is preferred to increase stability, a hydrophilic polymer selected from polyvinylpyrrolidone, polyvinyl alcohol, polyacrylic acid or a derivative thereof, hyaluronic acid or a salt thereof or polyethylene oxide, but preferably polyvinylpyrrolidone to increase hydrophilicity and lubricity, and an isocyanate-terminated prepolymer selected from polyoxyethylene-based isocyanate prepolymers such as a toluene or isophorone diisocyanate-based prepolymer and preferably Hypol* PreMA G60 manufactured by Hampshire Corporation, Lexington, Massachusetts, to increase binding strength. For further examples of suitable polyisocyanates see Encyclopedia of Polymer Science and Technology, H. F. Mark, N. G. Gaylord and N. M. Bikeles (eds.) (1969).

The lubricant coating of the present invention is generally prepared by first obtaining a mixing vat in which to prepare the solution. The mixing vat should be dry and free of water and alcohol. The present lubricant coating composition is preferably blended at room temperature according to the following component ratios described as follows in weight percent; i.e., 1 to 4 weight percent but preferably 1.9 weight percent polyvinylpyrrolidone, 0.5 to 3 weight percent but preferably 1.1 weight percent of the polyoxyethylene-based isocyanate-terminated prepolymer, 15 to 25 weight percent but preferably 18 weight percent alkylester of a carboxylic acid and 60 to 80 weight percent but preferably 79 weight percent toluene. The solution is mixed thoroughly until the polyvinylpyrrolidone and the prepolymer are completely dissolved. The component blending requires approximately 20 to 40 minutes but most usually about 30 minutes. The resulting lubricant coating solution should appear crystal clear with light yellow color. Prior to coating medical devices with the present lubricant coating solution, the particular medical device, such as a catheter, should for best results be cleaned by first filling a container with 100% isopropanol. The medical device is then dip washed in the isopropanol for approximately 5 seconds and dried by forced air at approximately 50 to 90° C to remove surface residual isopropanol and debris. The device should at this point be completely isopropanol free. The medical device is then dip coated for about 2 to 15 seconds in the lubricant coating solution while it is still warm to hot from the forced air drying and slowly removed from the solution vat at a rate of about 1 to 4 centimeters per second. The catheter or other medical device is then air dried at room temperature and less than about 40 percent humidity but preferably between 20 to 30 percent humidity for about 2 to 30 seconds but preferably 3 to 5 seconds to allow any excess lubricant coating solution to drain off. Optionally, excess lubricant may also be removed using absorbent towels. After air drying, the coated medical devices are baked in forced air ovens at approximately 70° C to 120° C preferably at 80° C for approximately 30 minutes to 3 hours but most preferably for one hour and then removed from the oven. The medical devices are preferably checked for adequate transparency and to ensure that no solvent smell is present.

In packaging the subject medical devices coated in accordance with the present invention, the devices should not be allowed to touch one another if the humidity is above about 60 percent. High humidity should likewise be avoided in that it could cause undesirable moisture absorption by the lubricant coating. To prevent or avoid contact between the coated medical devices, each device may be packed in either paper, polyethylene tubing or the like depending on the shape of the particular device. If necessary, due to high atmospheric humidity, a desiccant may likewise be necessary in the packaging.

The preferred method of making and using the lubricant coating of the present invention is described in even greater detail in the following examples which are provided for purposes of further illustration. The following examples as described are not intended to be construed as limiting the scope of the present invention.

### Example 1:

A lubricious coating was prepared by blending at room temperature the following components in a mixing vat for approximately 30 minutes until fully dissolved to form a crystal clear solution with a light yellow color.

| | wt% |
|---|---|
| Polyvinylpyrrolidone | 2.0 |
| Ethyl lactate | 18.0 |
| Toluene | 77.8 |
| Isocyanate-terminated prepolymer | 1.2 |

### Example 2:

Thoracic catheters made of polyvinyl chloride (PVC) were washed with isopropanol and dried at 80° C for 30 minutes. The catheters were dipped coated for 10 seconds while the catheters were still warm. After dipping, excessive coating was removed using paper towels. The catheters were then immediately baked at 80° C for 60 minutes. The resultant coating was transparent and colorless with good bonding. The coating was very lubricious when wet. The friction reduction was up to 60%.

### B. Biocompatibility tests

The coated catheters were first tested for hemolysis and then tested for lubricity using 1) protein adsorption and 2) platelet adhesion. The results of these tests show no hemolysis and improved lubricity as noted by a reduction of protein adsorption and platelet adhesion by more than 90% as set forth below.

### 1. Hemolysis

Using protein electrophoresis, no hemoglobin was seen in the supernatant of the PVP-coated thoracic catheter, nor was any seen in the hemolysis-negative control sample. As a comparison, rabbit hemoglobin was seen in gels stained with rabbit hemoglobin and the hemolysis-positive control. The results imply that the hydrophilic coating showed no sign of causing hemolysis.

### 2. Lubricity

### a) Protein (fibrinogen)adsorption

Table 1 summarizes protein adsorption on the subject coated catheter surfaces and that of the control samples.

**Table 1**

| material | n | ng | ng/cm²" |
|---|---|---|---|
| control | 4 | 1620±69 | 339.3±14.4 |
| PVP-coated | 4 | 87.6±27.1 | 18.3±5.7 |

| | | | |
|---|---|---|---|
| ": ID=0.4 cm, total surface area of 3.8 cm tubing =4.7cm² | | | |

Test results show the adsorption of fibrinogen onto the hydrophilic surface was decreased by more than 90% compared to control surface.

### b) Platelet adhesion

Table 2 summarizes platelet adhesion on the subject coated catheter surfaces and that of the control samples:

**Table 2**

| | | |
|---|---|---|
| material | n | Platelet/cm²(x10⁵) |
| control | 4 | 7.246±0.052 |
| PVP-coated | 4 | 0.055±0.019 |

Table 2 illustrates the platelet adhesion on both the PVP-coated and control catheters. The hydrophilic coating reduced platelet adhesion by more than 95%. The difference in platelet adhesion was further characterized by Scanning Electron Microscope (SEM) which showed control catheters having numerous platelets attached to the surface thereof while the subject PVP-coated catheters showed little sign of platelet adhesion.

The subject lubricant composition prepared in accordance with the present invention may be applied as a thin surface film, e.g., less than about 4.0 mill but most preferably less than about 2.5 mill in thickness, which upon contact with water or fluid sufficiently reduces the coefficient of friction to aid in the in vivo placement of medical devices.

The unexpected significant advantages of the present lubricious coating achieved through the particular composition formulation noted above include decreased wet coefficient of friction, decreased adherence with various surfaces and resistance to wet abrasion.

Medical devices once coated with the subject lubricious coating are to be packaged and sterilized using an appropriate sterilization technique or may be sterilized and then packaged using aseptic technique. Appropriate methods of sterilization and packaging are known to those skilled in the art and include gamma radiation, electronic beam, ethylene oxide, and like methods. Preferably, medical devices coated with the subject lubricious coating are packaged and then sterilized using gamma radiation by cobalt 60 with 1 to 4 mrads but preferably 2 mrads in two independent exposure cycles for superior results.

Appropriate packaging for the subject coated medical devices includes metallic foil pouches such as aluminum foil pouches, polyethylene film, ethylene vinyl acetate film, polypropylene film, polyvinyl chloride film and like packages known to those skilled in the art, but preferably, a polypropylene tube package or a package having a fibrous layer and a plastic layer with a pocket formed therebetween for storage of the coated medical device.

The method of using the subject coated medical devices comprises removing the device from its packaging, applying moisture to the lubricated surface of the device and placing the device as necessary for a particular medical procedure.

It is seen therefore that the present lubricious coating for medical devices provides an effective wet abrasion resistant, low coefficient of friction coating for medical devices. The lubricious coating, the method of making and using the lubricious coating, the coated medical devices and the method of using the coated medical devices as disclosed and described herein have specific advantages over the heretofore known lubricants for medical devices. The subject lubricious coating resists wet abrasion, adheres to a variety of surfaces, has a decreased coefficient of friction only when wetted and is biocompatible. Hence for these reasons as well as others, some of which hereinabove set forth, it is seen that the present lubricious coating represents a significant advancement in the art which has substantial commercial significance.

While there is shown and described herein certain specific embodiments of the invention, it will be manifest to those skilled in the art that various modifications may be made without departing from the scope of the underlying inventive concept and that the same is not limited to the particular forms herein shown and described except insofar as indicated by the scope of the appended claims.

## Claims

1. A lubricant composition comprising an alkylbenzene selected from the group consisting of toluene, xylene and styrene, a hydrophilic polymer selected from the group consisting of polyvinylpyrrolidone, polyvinyl alcohol, polyacrylic acid or a derivative thereof, hyaluronic acid or a salt thereof and polyethylene oxide, an isocyanate-terminated prepolymer selected from the group consisting of polyoxyethylene-based isocyanate prepolymers and an alkylester of a carboxylic acid selected from the group consisting of ethyl lactate, methylbenzoate and propylacrylate.

2. The composition of claim 1 wherein said alkylbenzene is toluene.

3. The composition of claim 1 wherein said hydrophilic polymer is polyvinylpyrrolidone.

4. The composition of claim 1 wherein said alkylester of a carboxylic acid is ethyl lactate.

5. The composition of claim 1 wherein said isocyanate-terminated prepolymer is selected from the group consisting of toluene and isophorone diisocyanate-based prepolymers.

6. The lubricant composition according to claim 1 comprising 1 to 4 weight percent polyvinylpyrrolidone, 15 to 25 weight percent ethyl lactate, 60 to 80 weight percent toluene and 0.5 to 3 weight percent isocyanate-terminated prepolymer.

7. Use of the composition of anyone of claims 1 to 6 as a lubricant coating for medical devices.

8. A method for producing a lubricant composition according to anyone of claims 1 to 6 comprising blending an alkylbenzene selected from the group consisting of toluene, xylene and styrene, a hydrophilic polymer selected from the group consisting of polyvinylpyrrolidone, polyvinyl alcohol, polyacrylic acid or a derivative thereof, hyaluronic acid or a salt thereof and polyethylene oxide, an isocyanate-terminated prepolymer selected from the group consisting of polyoxyethylene-based isocyanate prepolymers and an alkylester of a carboxylic acid selected from the group consisting of ethyl lactate, methylbenzoate and propylacrylate until dissolved.

9. The method of claim 8 comprising blending polyvinylpyrrolidone, ethyl lactate, toluene and isocyanate-terminated prepolymer until dissolved.

10. A medical device at least partially coated with a lubricant composition according to anyone of claims 1 to 6.

11. A method of coating at least a portion of a medical device with a lubricious coating comprising:
washing the medical device;
drying the medical device;
applying a lubricious composition according to anyone of claims 1 to 6;
removing excess lubricious coating; and
baking the medical device.

12. The method of claim 11 wherein said medical device is a catheter.

13. The method of claim 11 wherein said medical device is selected from the group consisting of catheters, arterial venous shunts, gastroenteric feed tubes and endotracheal tubes.

14. The method of claim 11 wherein said washing comprises dipping the medical device in isopropanol.

15. The method of claim 11 wherein said drying comprises exposing the medical device to forced air of 50 to 90 degrees Celsius.

16. The method of claim 11 wherein said drying comprises exposing the medical device to forced air of 80 degrees Celsius.

17. The method of claim 11 wherein said applying of the lubricious coating comprises dipping a warm medical device into the coating for about 2 to 15 seconds.

18. The method of claim 11 wherein said removing of excess coating comprises drying the medical device at room temperature.

19. The method of claim 11 wherein said removing of excess coating comprises removal with absorbent towels.

## Patentansprüche

1. Eine Schmiermittelzusammensetzung umfassend ein Alkylbenzol ausgewählt aus der Gruppe bestehend aus Toluol, Xylol und Styrol, ein hydrophiles Polymer ausgewählt aus der Gruppe bestehend aus Polyvinylpyrrolidon, Polyvinylalkohol, Polyacrylsäure oder einem Derivat derselben, Hyaluronsäure oder einem Salz desselben und Polyethylenoxid, ein isocyanatterminiertes Vorpolymer ausgewählt aus der Gruppe bestehend aus polyoxyethylenbasierten Isocyanatvorpolymeren, und einen Alkylester einer Carbonsäure ausgewählt aus der Gruppe bestehend aus Ethyllactat, Methylbenzoat und Propylacrylat.

2. Die Zusammensetzung gemäss Anspruch 1, worin das Alkylbenzol Toluol ist.

3. Die Zusammensetzung gemäss Anspruch 1, worin das hydrophile Polymer ein Polyvinylpyrrolidon ist.

4. Die Zusammensetzung gemäss Anspruch 1, worin der Alkylester einer Carbonsäure Ethyllactat ist.

5. Die Zusammensetzung gemäss Anspruch 1, worin das isocyanatterminierte Vorpolymer ausgewählt ist aus der Gruppe bestehend aus Toluol und isophorondiisocyanatbasierten Vorpolymeren.

6. Die Schmiermittelzusammensetzung gemäss Anspruch 1 umfassend 1 bis 4 Gewichtsprozent Polyvinylpyrrolidon, 15 bis 25 Gewichtsprozent Ethyllactat, 60 bis 80 Gewichtsprozent Toluol und 0.5 bis 3 Gewichtsprozent isocyanatterminiertes Vorpolymer.

7. Verwendung der Zusammensetzung gemäss irgendeinem der Ansprüche 1 bis 6 als eine Schmiermittelbeschichtung für medizinische Vorrichtungen.

8. Ein Verfahren zur Herstellung einer Schmiermittelzusammensetzung gemäss einem der Ansprüche 1 bis 6 umfassend das Mischen eines Alkylbenzols ausgewählt aus der Gruppe bestehend aus Toluol, Xylol und Styrol, eines hydrophilen Polymers ausgewählt aus der Gruppe bestehend aus Polyvinylpyrrolidon, Polyvinylalkohol, Polyacrylsäure oder einem Derivat derselben, Hyaluronsäure oder einem Salz desselben und Polyethylenoxid, eines isocyanatterminierten Vorpolymers ausgewählt aus der Gruppe bestehend aus polyoxyethylenbasierten Isocyanatvorpolymeren, und eines Alkylesters einer Carbonsäure ausgewählt aus der Gruppe bestehend aus Ethyllactat, Methylbenzoat und Propylacrylat, bis diese gelöst sind.

9. Das Verfahren gemäss Anspruch 8 umfassend das Mischen von Polyvinylpyrrolidon, Ethyllactat, Toluol und isocyanatterminiertem Vorpolymer bis diese gelöst sind.

10. Eine mindestens teilweise mit einer Schmiermittelzusammensetzung gemäss einem der Ansprüche 1 bis 6 beschichtete medizinische Vorrichtung.

11. Ein Verfahren zur Beschichtung mindestens eines Teils einer medizinischen Vorrichtung mit einer Schmierbeschichtung umfassend:
Waschen der medizinischen Vorrichtung;
Trocknen der medizinischen Vorrichtung;
Auftragen einer Schmierzusammensetzung gemäss einem der Ansprüche 1 bis 6;
Entfernen von überschüssiger Schmierbeschichtung; und Backen der medizinischen Vorrichtung.

12. Das Verfahren gemäss Anspruch 11, worin die medizinische Vorrichtung ein Katheter ist.

13. Das Verfahren gemäss Anspruch 11, worin die medizinische Vorrichtung ausgewählt ist aus der Gruppe bestehend aus Kathetern, arteriellvenösen Shunts, gastroenteralen Ernährungsröhren und endotrachealen Röhren.

14. Das Verfahren gemäss Anspruch 11, worin das Waschen Eintauchen der medizinischen Vorrichtung in Isopropanol umfasst.

15. Das Verfahren gemäss Anspruch 11, worin das Trocknen das Aussetzen der medizinischen Vorrichtung einer Druckluft von 50 bis 90 Grad Celsius umfasst.

16. Das Verfahren gemäss Anspruch 11, worin das Trocknen Aussetzen der medizinischen Vorrichtung einer Druckluft von 80 Grad Celsius umfasst.

17. Das Verfahren gemäss Anspruch 11, worin das Auftragen der Schmierbeschichtung das Eintauchen einer warmen medizinischen Vorrichtung in die Beschichtung während etwa 2 bis 15 Sekunden umfasst.

18. Das Verfahren gemäss Anspruch 11, worin das Entfernen überschüssiger Beschichtung Trocknen der medizinischen Vorrichtung bei Raumtemperatur umfasst.

19. Das Verfahren gemäss Anspruch 11, worin das Entfernen von überschüssiger Beschichtung die Entfernung mit absorbierenden Handtüchern umfasst.

## Revendications

1. Composition de lubrifiant comprenant un alkylbenzène choisi dans le groupe consistant en le toluène, le xylène et le styrène, un polymère hydrophile choisi dans le groupe consistant en la polyvinylpyrrolidone, l'alcool polyvinylique, l'acide polyacrylique ou un de ses dérivés, l'acide hyaluronique ou un de ses sels et le poly(oxyde d'éthylène), un prépolymère à terminaison isocyanate choisi dans le groupe consistant en des prépolymères d'isocyanate à base de polyoxyéthylène, et un ester alkylique d'un acide carboxylique choisi dans le groupe consistant en le lactate d'éthyle, le benzoate de méthyle et l'acrylate de propyle.

2. Composition suivant la revendication 1, dans laquelle ledit alkylbenzène est le toluène.

3. Composition suivant la revendication 1, dans laquelle ledit polymère hydrophile est la polyvinylpyrrolidone.

4. Composition suivant la revendication 1, dans laquelle ledit ester alkylique d'un acide carboxylique est le lactate d'éthyle.

5. Composition suivant la revendication 1, dans laquelle ledit prépolymère à terminaison isocyanate est choisi dans le groupe consistant en des prépolymères à base de toluène-diisocyanate et d'isophorone-diisocyanate.

6. Composition de lubrifiant suivant la revendication 1, comprenant 1 à 4 % en poids de polyvinylpyrrolidone, 15 à 25 % en poids de lactate d'éthyle, 60 à 80 % en poids de toluène et 0,5 à 3 % en poids de prépolymère à terminaison isocyanate.

7. Utilisation de la composition suivant l'une quelconque des revendications 1 à 6 comme revêtement lubrifiant pour des dispositifs médicaux.

8. Procédé pour la production d'une composition de lubrifiant suivant l'une quelconque des revendications 1 à 6, comprenant l'étape consistant à mélanger un alkylbenzène choisi dans le groupe consistant en le toluène, le xylène et le styrène, un polymère hydrophile choisi dans le groupe consistant en la polyvinylpyrrolidone, l'alcool polyvinylique, l'acide polyacrylique ou un de ses dérivés, l'acide hyaluronique ou un de ses sels et le poly(oxyde d'éthylène), un prépolymère à terminaison isocyanate choisi dans le groupe consistant en des prépolymères d'isocyanate à base de polyoxyéthylène, et un ester alkylique d'un acide carboxylique choisi dans le groupe consistant en le lactate d'éthyle, le benzoate de méthyle et l'acrylate de propyle jusqu'à leur dissolution.

9. Procédé suivant la revendication 8, comprenant l'étape consistant à mélanger de la polyvinylpyrrolidone, du lactate d'éthyle, du toluène et un prépolymère à terminaison isocyanate jusqu'à leur dissolution.

10. Dispositif médical revêtu au moins partiellement avec une composition de lubrifiant suivant l'une quelconque des revendications 1 à 6.

11. Procédé pour revêtir au moins une partie d'un dispositif médical avec un revêtement lubrifiant, consistant :
à laver le dispositif médical ;
à sécher le dispositif médical ;
à appliquer une composition lubrifiante suivant l'une quelconque des revendications 1 à 6 ;
à éliminer l'excès de revêtement lubrifiant ; et
à soumettre le dispositif médical à une cuisson.

12. Procédé suivant la revendication 11, dans lequel ledit dispositif médical est un cathéter.

13. Procédé suivant la revendication 11, dans lequel ledit dispositif médical est choisi dans le groupe consistant en des cathéters, des dérivations artério-veineuse, des tubes de sondes gastro-entériques et des tubes endotrachéaux.

14. Procédé suivant la revendication 11, dans lequel ledit lavage comprend l'immersion du dispositif médical dans de l'isopropanol.

15. Procédé suivant la revendication 11, dans lequel ledit séchage comprend l'exposition du dispositif médical à de l'air forcé à une température de 50 à 90 degrés Celsius.

16. Procédé suivant la revendication 11, dans lequel ledit séchage comprend l'exposition du dispositif médical à de l'air forcé à une température de 80 degrés Celsius.

17. Procédé suivant la revendication 11, dans lequel ladite application du revêtement lubrifiant comprend l'immersion d'un dispositif médical chaud dans le revêtement pendant environ 2 à 15 secondes.

18. Procédé suivant la revendication 11, dans lequel ladite élimination de l'excès de revêtement comprend le séchage du dispositif médical à température ambiante.

19. Procédé suivant la revendication 11, dans lequel ladite élimination de l'excès de revêtement comprend l'élimination avec des serviettes absorbantes.
